(19) Europäisches Patentamt
European Patent Office
Office européen des brevets

(11) EP 0 569 294 B1

(12) **FASCICULE DE BREVET EUROPEEN**

(45) Date de publication et mention de la délivrance du brevet:
**17.07.1996 Bulletin 1996/29**

(51) Int. Cl.$^6$: **C07C 311/53**, A61K 7/043

(21) Numéro de dépôt: **93401156.0**

(22) Date de dépôt: **05.05.1993**

(54) **Utilisation d'un arylsulfonyluréthanne comme résine garnissante dans les vernis à ongles nitrocellulosiques. Nouveaux arylsulfonyluréthannes et nouveaux vernis à ongles nitrocellulosiques**

Anwendung eines Arylsulfonylurethans als verstärkendes Harz in cellulosem Nagellackmittel-Arylsulfonylurethane und cellulose Nagellackmittel

Use of an arylsulfonylurethane as strengthening resin in nitrocellulose nail lacquers, arylsulfonylurethanes and nitrocellulose nail lacquers

(84) Etats contractants désignés:
**BE DE ES FR GB IT LU**

(30) Priorité: **06.05.1992 FR 9205560**

(43) Date de publication de la demande:
**10.11.1993 Bulletin 1993/45**

(73) Titulaire: **SOCIETE NATIONALE DES POUDRES ET EXPLOSIFS**
**F-75181 Paris Cédex 04 (FR)**

(72) Inventeurs:
- **Lecacheur, Maryse**
  **F24100 Bergerac (FR)**
- **Mutterer, Vincent**
  **F-67000 Strasbourg (FR)**
- **Wimmer, Eric**
  **F-45800 Saint Jean de Braye (FR)**

(74) Mandataire: **Pech, Bernard et al**
**Sté Nationale des Poudres et Explosifs**
**12, quai Henri IV**
**75181 Paris Cédex 04 (FR)**

(56) Documents cités:

| | |
|---|---|
| EP-A- 0 449 488 | WO-A-90/12829 |
| DE-A- 3 919 747 | FR-A- 1 239 917 |
| FR-A- 1 340 297 | FR-A- 1 471 089 |
| FR-A- 2 326 414 | US-A- 3 607 935 |
| US-A- 3 687 934 | US-A- 3 787 491 |
| US-A- 3 933 894 | US-A- 4 513 127 |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.12.4/3.4

## Description

La présente invention se situe dans le domaine des vernis à ongles nitrocellulosiques, incolores ou colorés. Elle concerne plus particulièrement le domaine des vernis à ongles essentiellement constitués de nitrocellulose, d'une résine garnissante, d'un plastifiant, d'un agent gélifiant et d'un système solvant-diluant. Elle concerne également de nouveaux arylsulfonyluréthannes.

Un vernis à ongles nitrocellulosique coloré est en général obtenu par mélange d'une base antisédimentante nitrocellulosique thixotrope incolore, qui est en fait un vernis à ongles incolore, avec un ou des pigments ou colorants, ou avec une base colorante qui peut être par exemple obtenue par mélange d'une base antisédimentante avec un ou des pigments.

La base antisédimentante nitrocellulosique thixotrope incolore est, selon un procédé traditionnel, obtenue par mélange d'un gel nitrocellulosique thixotrope incolore et d'une base incolore non thixotrope, de préférence en présence d'une faible quantité d'un acide tel que l'acide phosphorique ou l'acide citrique pour favoriser la thixotropie de la base antisédimentante.

La base incolore non thixotrope est constituée de nitrocellulose, d'une résine garnissante, d'un plastifiant et de solvants et/ou diluants.

Le gel nitrocellulosique thixotrope incolore est constitué de nitrocellulose, d'un agent gélifiant, d'un plastifiant, de solvants et/ou diluants et éventuellement d'un agent mouillant.

Les principales caractéristiques que doivent présenter les vernis à ongles sont bien connues de l'homme du métier. Ils doivent notamment être inoffensifs pour la santé.

La résine garnissante utilisée dans les vernis à ongles nitrocellulosiques permet d'apporter du garnissant au vernis, autrement dit d'augmenter l'extrait sec, de manière à ce que la quantité de liant déposée après évaporation soit la plus grande possible. La résine contribue également au brillant du vernis et à l'adhérence sur l'ongle.

Au stade industriel, les résines arylsulfonamideformaldéhyde, qui présentent de nombreux avantages, sont pratiquement seules utilisées. Elles ont toutefois l'inconvénient majeur de libérer du formol au cours du temps. Or, il s'avère que le formol est un composé cancérigène et, de ce fait, l'homme du métier est à la recherche d'une nouvelle résine garnissante, compatible avec les autres constituants des vernis à ongles nitrocellulosiques, ayant les avantages des résines arylsulfonamide-formol jusqu'alors utilisées, mais qui ne présenterait pas l'inconvénient précité.

L'état de la technique propose quelques solutions.

La demande de brevet PCT WO 90/12829 décrit l'utilisation de résines époxysulfonamides dans les compositions pour cosmétiques et notamment dans les vernis à ongles nitrocellulosiques. Ces résines sont toutefois d'un coût prohibitif pour une utilisation industrielle.

La demande de brevet français FR 2 421 604 décrit des vernis à ongles nitrocellulosiques comprenant, comme produit de substitution aux résines arylsulfonamide-formol, une composition comprenant le benzoate de saccharose seul ou en mélange avec le méthacrylate de méthyle, de l'acétate-isobutyrate de saccharose et un phtalate, un adipate ou un phosphate organique. Cette solution nécessite donc la réalisation préalable d'un prémélange, ce qui rend le procédé plus complexe et onéreux.

Les solutions connues ne sont donc pas totalement satisfaisantes.

La présente invention a pour objet une solution au problème précité du remplacement des résines arylsulfonamide-formol dans les vernis à ongles nitrocellulosiques, solution qui ne présente pas les inconvénients précités des solutions jusqu'alors proposées.

De façon inattendue, la Demanderesse a constaté que l'utilisation d'arylsulfonyluréthannes comme résine garnissante dans les vernis à ongles nitrocellulosiques à la place des résines arylsulfonamide-formol permettait, de façon simple et peu coûteuse, d'obtenir des vernis à ongles ayant des propriétés voisines et satisfaisantes, sans formation de formol au cours du temps.

On entend par "arylsulfonyluréthannes" des composés organiques comportant un -les mono(arylsulfonyluréthannes)-ou plusieurs -les poly(arylsulfonyluréthannes)-groupement arylsulfonyluréthanne de structure

$$ArSO_2NH-\overset{\overset{O}{/\!/}}{C}-O-,$$

Ar désignant un groupement aromatique.

De nombreux mono(arylsulfonyluréthannes) sont connus. Le brevet US 4 287 083 décrit par exemple l'utilisation d'uréthannes acyloines, notamment de

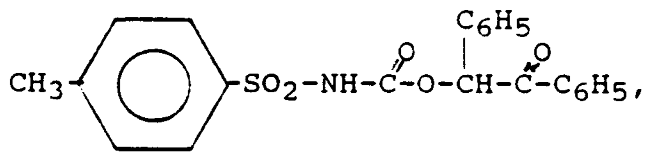

comme photoinitiateurs de polymérisation radicalaire.

D'autres mono(arylsulfonyluréthannes) sont connus comme dérivés phytosanitaires ou comme intermédiaires de synthèse de médicaments.

Le brevet US 4 513 127 enseigne par ailleurs l'utilisation des poly(arylsulfonyluréthannes) comme accélérateurs de polymérisation de monomères acryliques réticulables. Ce brevet décrit des di(arylsulfonyluréthannes) ayant un squelette polymérique silane ou organosiloxane de masse 1860 ou 5360.

La présente invention a donc pour objet de nouveaux poly(arylsulfonyluréthannes), comprenant de 2 à 5, c'est à dire 2, 3, 4 ou 5, groupements arylsulfonyluréthannes, ayant une masse moléculaire comprise entre 450 et 1500, et de façon particulièrement préférée ceux dont le groupement aryle est le groupement paratolyle

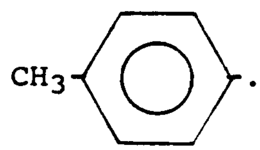

Selon une variante préférée, les poly(arylsulfonyluréthannes) selon l'invention sont caractérisés en ce qu'ils répondent à la formule générale (II)

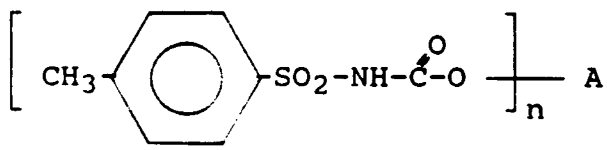

dans laquelle n représente un nombre entier tel que 2 ≤ n ≤ 5 et A représente un radical organique, divalent lorsque n = 2, trivalent lorsque n = 3, tétravalent lorsque n = 4 et pentavalent lorsque n = 5, qui est le squelette de polyols de structure $A(OH)_n$ choisis dans le groupe constitué par :

- les polyméthylèneglycols, par exemple le glycol et le triméthylèneglycol,
- les polyoxyalkylèneglycols, par exemple le dioxyéthylèneglycol, le trioxyéthylèneglycol, le dioxypropylèneglycol et le trioxypropylèneglycol,
- les polyéthers triols et les polyéthers tétraols,
- le glycérol et le xylitol,
- les triméthylolalcanes, par exemple le triméthylolpropane.

Comme polyéthers triols, on préfère ceux répondant aux formules générales :

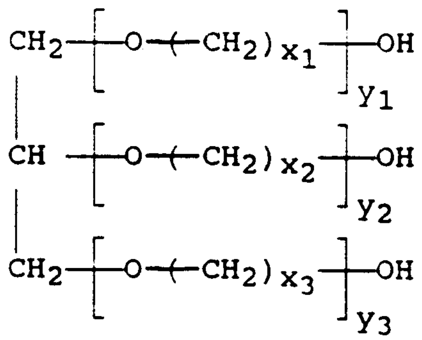

ou

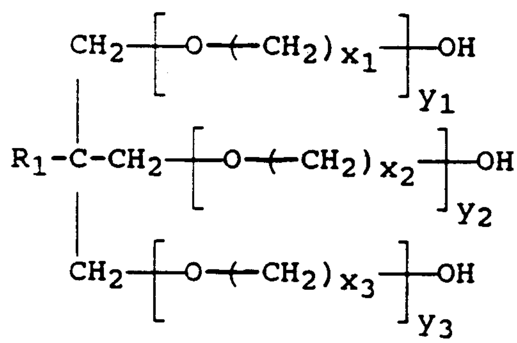

dans lesquelles

- . $x_1$, $x_2$ et $x_3$, identiques ou différents, de préférence identiques, représentent 1, 2, 3 ou 4,
- . $y_1$, $y_2$ et $y_3$, identiques ou différents, représentent 0, 1, 2, 3, 4, 5 ou 6 à la condition que $y_1$, $y_2$ et $y_3$ ne soient pas simultanément nuls,
- . $R_1$ représente l'hydrogène ou une chaîne alkyle, linéaire ou ramifiée, comportant 1 à 8 atomes de carbone, ou une chaine alkyle comportant 1 à 8 atomes de carbone substituée par au moins un radical alcoxy.

De préférence $x_1 = x_2 = x_3 = 2$ et $y_1$, $y_2$ et $y_3$ représentent 2 ou 3.

Comme polyéthers tétraols, on préfère ceux répondant à la formule générale

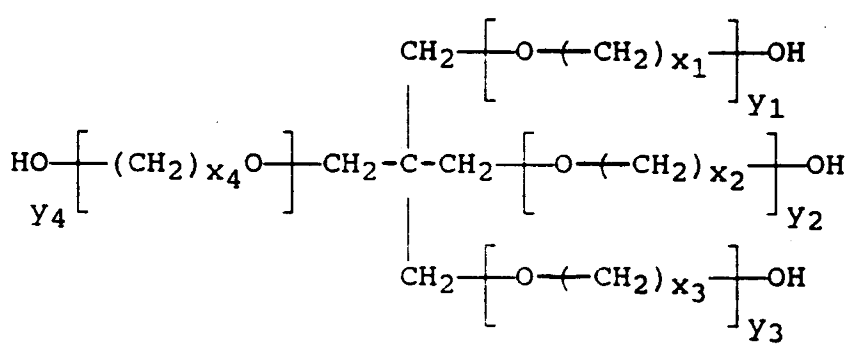

dans laquelle

- . $x_1$, $x_2$, $x_3$ et $x_4$, identiques ou différents, de préférence identiques, représentent 1, 2, 3 ou 4,
- . $y_1$, $y_2$, $y_3$ et $y_4$, identiques ou différents, représentent 0, 1, 2, 3, 4, 5 ou 6 à la condition que $y_1$, $y_2$, $y_3$ et $y_4$ ne soient pas simultanément nuls.

De préférence $x_1 = x_2 = x_3 = x_4 = 2$ et $y_1$, $y_2$, $y_3$ et $y_4$ représentent 1, 2 ou 3.

Les arylsulfonyluréthannes peuvent par exemple être obtenus par réaction d'un arylsulfonylisocyanate avec un alcool.

Les poly(arylsulfonyluréthannes) selon l'invention peuvent être obtenus par réaction de Ar-$SO_2$-NCO avec un polyol, Ar désignant un groupement aromatique, éventuellement hétérocyclique, par exemple un groupement phényle ou naphtyle, non substitué, ou substitué par exemple par une chaîne alkyle $C_1$ à $C_4$. De façon particulièrement préférée, Ar représente le groupement paratolyle. Chaque hydroxyle du polyol réagissant avec une fonction isocyanate, cela permet l'obtention d'un nombre de groupements arylsulfonyluréthannes identique au nombre de fonctions hydroxyles du polyol.

Les composés de départ, notamment

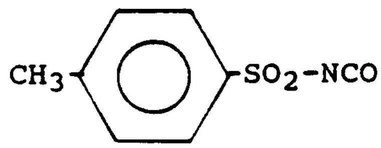

et la plupart des polyols A $(OH)_n$ précités sont des produits industriels usuels et peu coûteux. Par ailleurs la réaction entre l'isocyanate et l'alcool est simple de mise en oeuvre et peu onéreuse.

Les poly(arylsulfonyluréthannes) selon l'invention sont donc des produits très bon marché et très facilement accessibles.

Lors de la réaction entre l'arylsulfonylisocyanate et l'alcool, selon une variante préférée, on agite le milieu réactionnel et on contrôle la température, qu'on fait varier en fonction de la viscosité du milieu, afin d'éviter une prise en masse intempestive.

On préfère également travailler sous gaz inerte, azote par exemple.

Selon une autre variante préférée, on travaille sans solvant mais il est également possible de travailler en milieu solvant, diméthylformamide (DMF) par exemple, lorsque le milieu est très visqueux.

Selon une autre variante préférée, on ajoute progressivement l'isocyanate à l'alcool placé dans le réacteur et l'on opère avec un rapport fonctionnel NCO/OH compris entre 0,99 et 1,00. La fin de réaction peut être contrôlée par dosage des fonctions isocyanates résiduelles dans le milieu réactionnel.

On peut également accélérer la cinétique de la réaction on opérant en présence d'un catalyseur usuel de réactions isocyanates-alcools.

La mise en oeuvre de ce procédé est très simple, le produit recherché, en général solide ou liquide visqueux à la température ambiante, pouvant être directement obtenu sans étape supplémentaire de concentration et/ou de purification, par simple mélange des deux composés de départ.

La présente invention a également pour objet de nouveaux vernis à ongles nitrocellulosiques, plus précisément ceux essentiellement constitués de nitrocellulose, d'une résine garnissante, d'un plastifiant, d'un agent gélifiant et d'un système solvant/diluant. Ces vernis sont caractérisés en ce que la résine garnissante comprend un arylsulfonyluréthanne.

L'emploi du singulier pour désigner les constituants ne doit pas être pris dans un sens limitatif, la nitrocellulose pouvant être un mélange de diverses nitrocelluloses, la résine un mélange de plusieurs résines, etc.

L'expression "essentiellement constitué de" signifie que les constituants cités sont des constituants dont la présence est indispensable et qu'ils sont globalement majoritaires dans le vernis à ongles, qui peut par ailleurs comprendre des additifs usuels, variables selon les divers types de vernis, tels que pigments, colorants, agents mouillants, agents gonflants, essences d'Orient et agents absorbant les rayonnements UV.

La résine garnissante qui, selon l'invention, comprend un arylsulfonyluréthanne, peut également comprendre une ou plusieurs résines jusqu'alors utilisées, par exemple une résine arylsulfonamide-formol, une résine alkyde ou une resine acrylique. Toutefois, de façon préférée, la resine garnissante est uniquement constituée d'un ou de plusieurs arylsulfonyluréthannes.

Comme arylsulfonyluréthannes, on préfère utiliser les nouveaux arylsulfonyluréthannes précites selon l'invention, et, de façon particulière, les sous-groupes précités preferes de ces composés.

Toutefois, lorsqu'on utilise néanmoins un mono-(arylsulfonyluréthanne), on préfère utiliser ceux de formule générale (I)

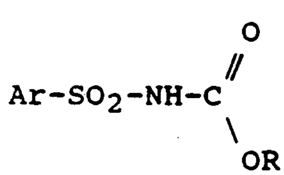

dans laquelle

. Ar a la signification précitée,

. R represente soit un groupement alkyle ou cycloalkyle, lineaire ou ramifie, soit un groupement alkyle ou cycloalkyle substitue par au moins un groupement alcoxy (par exemple methoxy, éthoxy, propoxy, butoxy) ou polyoxyalkyle. De façon préférée R comprend de 2 à 12 atomes de carbone.

Selon une variante préférée, les mono(arylsulfonyluréthannes) de formule (I) ont une masse moléculaire comprise entre 230 et 350.

De façon générale, les mono(arylsulfonyluréthannes), notamment ceux précités de formule (I), peuvent être obtenus comme précité pour les poly(arylsulfonyluréthannes) selon l'invention, mais en remplacant le polyol par un monoalcool.

Selon l'invention, la nitrocellulose peut comprendre toute nitrocellulose utilisée de façon générale comme agent filmogène dans les vernis à ongles classiques. On utilise par exemple la nitrocellulose "1/2 seconde" ou la nitrocellulose "1/4 seconde" selon la norme americaine, respectivement E33 et E27 selon la norme européenne, de masse moléculaire respectivement 36000 et 26000. On peut utiliser un mélange en proportions quelconques de diverses nitrocelluloses.

Le plastifiant a pour fonction de diminuer la durete du film en lui donnant de la souplesse. Certains esters, cetones et ethers peuvent être utilisés pour plastifier un vernis nitrocellulosique. Comme exemples de plastifiant utilisable pour mettre en oeuvre la présente invention on peut citer, sans que cette liste soit limitative, les composés suivants : adipate de diéthyle, adipate de dibutyle, adipate de diisobutyle, adipate de dihexyle, adipate de dicapryle, adipate de bis (2-éthylhexyle) , adipate de diisooctyle, adipate de dinonyle, adipate d'octyle et de décyle, adipate d'isooctyle et d'isodécyle, adipate de didecyle, adipate de diisodécyle, adipate d'isodécyle et d'octyle, adipate de polypropylèneglycol, adipate de bis (méthoxyéthyle), adipate de bis (éthoxyéthyle), adipate de bis (butoxyéthyle), adipate de bis-(butoxyéthoxyéthyle), phtalate de diméthyle, phtalate de diéthyle, phtalate de dipropyle, phtalate de dibutyle, phtalate de diisobutyle, phtalate de dihexyle, phtalate de butyle et d'octyle, phtalate de butyle et d'isodécyle, phtalate de butyle et d'isohexyle, phtalate de lauryle et d'isohexyle, phtalate de dioctyle, phtalate de diisooctyle, phtalate de dicapryle, phtalate de bis (2-éthylhexyle), phtalate de dinonyle, phtalate de bis (ethyldecyle), phtalate d'isooctyle et d'isodécyle, phtalate de didécyle, phtalate d'éthylhexyle et de décyle, phtalate de butyle et d'éthylhexyle, phtalate de bisméthoxyéthyle, phosphate de (2-éthylhexyle) et de diphényle, phosphate de triaryle modifie, phosphate de triphényle, phosphate d'isodécyle et de diphényle, le benzoate de benzyle, l'acétylricinoléate de butyle, l'acétylricinoléate de glyceryle, le glycolate de butyle, le stéarate de butyle, le citrate de diéthyle, le citrate de tributyle, l'acétylcitrate de tributyle, l'acétylcitrate de triéthyl-2 hexyle, le tartrate de di-butyle, ainsi que les mélanges de ces composés.

Les agents gélifiants préférés sont les argiles organophiles de type montmorillonite modifiée par une amine. On peut citer toutes celles décrites dans le brevet US 3 422 185 notamment celles commercialisées par National Lead Company sous la marque deposee "Bentone".

Le systeme solvant-diluant peut être aromatique, ou non aromatique.

Dans le cas d'un système non aromatique, il est par exemple constitué d'esters aliphatiques tels que l'acétate de butyle et l'acétate d'éthyle, utilisés comme solvants, et d'alcools aliphatiques tels que l'isopropanol et le butanol, utilisés comme diluants.

Dans le cas d'un systeme aromatique, il est en général constitué d'un ou de plusieurs des esters et alcools aliphatiques précités en mélange avec un hydrocarbure aromatique, par exemple le toluène.

Selon une variante préférée de l'invention, le vernis a ongles est constitué de 5 % à 20 % en poids de nitrocellulose, de 1 % à 20 % en poids d'une résine arylsulfonyluréthanne, de 0,1 % à 5 % en poids d'argile organophile de type montmorillonite modifiée par une amine, de 0,1 % à 20 % en poids de plastifiant, de 5 % à 85 % en poids d'un système solvant-diluant, de 0 a 10 % (de preference 0 % ou entre 1 % et 10 %) en poids de pigment ou colorant, et de 0 à 10 % (de preference 0 % ou entre 0,1 % et 10 %) en poids d'additifs usuels, la somme des pourcentages étant égale à 100.

Les vernis à ongles selon l'invention peuvent être obtenus selon la technologie précitée, à savoir par mélange d'un gel nitrocellulosique thixotrope incolore et d'une base incolore non thixotrope, ce qui permet d'obtenir une base antisédimentante nitrocellulosique thixotrope incolore, qui est un vernis à ongles incolore, dans laquelle on rajoute éventuellement des pigments et/ou colorants si l'on veut obtenir un vernis à ongles coloré. Selon l'invention, on remplace, dans ce procédé, tout ou partie de la résine garnissante habituellement utilisée par un arylsulfonyluréthanne, notamment par les nouveaux arylsulfonyluréthannes precites selon l'invention.

Les exemples non limitatifs suivants illustrent l'invention et les avantages qu'elle procure.

Exemple 1 Tosyluréthanne d'un polyéthertriol du glycérol de masse 400

Ce composé est obtenu par réaction de l'isocyanate de tosyle de formule

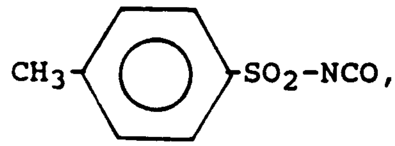

qui est un produit commercial industriel, avec le polyéthertriol du glycerol de masse 400 de formule suivante :

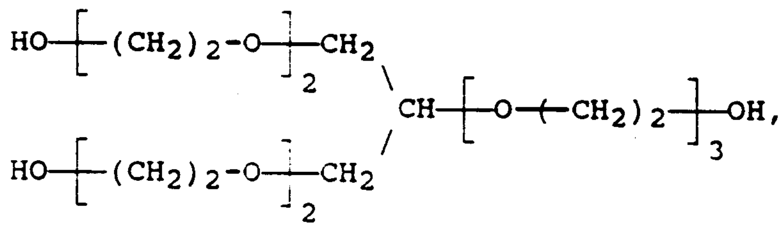

produit commercial pouvant être obtenu par réaction entre le glycérol et l'oxyde d'éthylène.

On utilise un réacteur à double enveloppe de 1 l. On contrôle la température à l'aide d'une circulation d'huile. Des sondes permettent la mesure de la température de l'huile et celle du mélange réactionnel. La réaction se fait sous un ledger courant d'azote. Le réacteur est surmonté d'une ampoule à brome contenant 140,8 g d'isocyanate de tosyle, l'azote arrivant sur l'isocyanate, d'un refrigerant ainsi que d'un agitateur mecanique permettant d'homogénéiser le milieu réactionnel. L'alcool (96,1 g), dont le teneur en fonction hydroxyle est de 7,43 eq/kg, est place dans le reacteur. On fixe la temperature initiale du bain d'huile a 30°C, puis on ajoute goutte a goutte l'isocyanate, en élevant la température du bain d'huile en fonction de la viscosite du milieu de façon a eviter sa prise en masse. Une fois l'addition terminee, on maintient l'agitation jusqu'à disparition totale de l'isocyanate tout en augmentant la température d'environ 10°C. Le suivi de l'isocyanate residuel est effectué par dosage sur des prelevements du milieu. La duree totale de réaction est environ 1,5 h et la température maximale atteinte est voisine de 60°C. On coule alors le produit (237 g) qui se presente sous forme d'une résine blanchâtre. A la température ambiante (20°C environ), cette résine est un liquide très visqueux. Le chromatogramme GPC (chromatographie par permeation de gel) avec double détection UV et réfractométrique montre que le produit de réaction est unique et que la réaction est quantitative.

Les spectres IR et RMN $^1H$ montrent par ailleurs que la résine a comme structure :

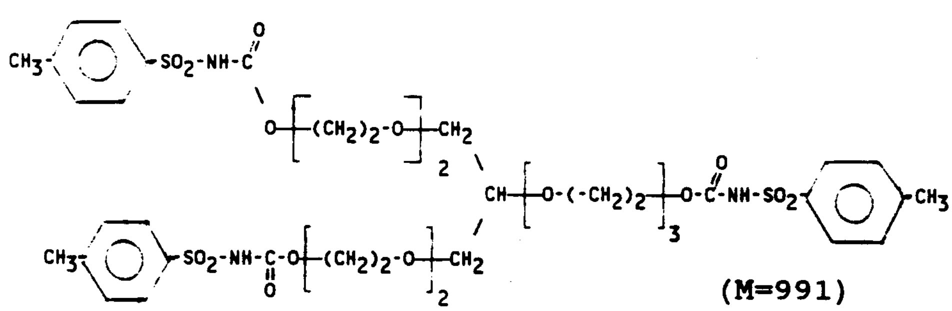

(M=991)

Exemple 2 Tosyluréthanne d'un polyéthertétraol du pentaérythritol de masse 576

On opère comme pour l'exemple 1 en utilisant, à la place du polyéthertriol du glycérol, 104,6 g du polyéthertétraol de masse 576 et de formule suivante :

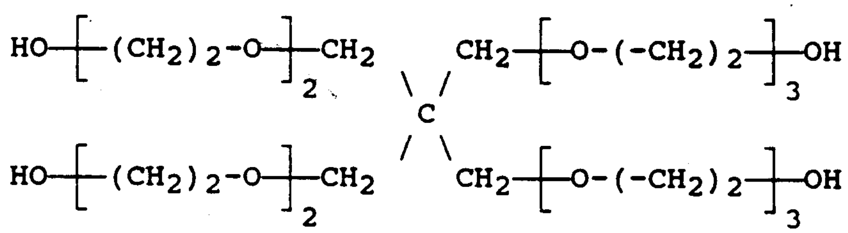

Ce polyéther tétraol est un produit commercial, pouvant être obtenu par réaction entre le pentaérythritol et l'oxyde d'éthylène. La teneur expérimentale en fonctions hydroxyles du composé utilisé est 6,54 eq/kg.

On utilise par ailleurs 135 g (0,685 mol) d'isocyanate de tosyle.

La durée totale de réaction est environ 1,3 h et la température maximale atteinte 96°C environ. A cette température, le produit obtenu (239,6 g) se présente sous forme d'une résine translucide. A la température ambiante (20°C environ) il se présente sous forme d'un solide non cristallisé, dur mais légèrement collant.

L'analyse par GPC montre que le produit de réaction est unique et que la réaction est quantitative.

Les spectres IR et RMN $^1$H montrent que cette résine a comme structure :

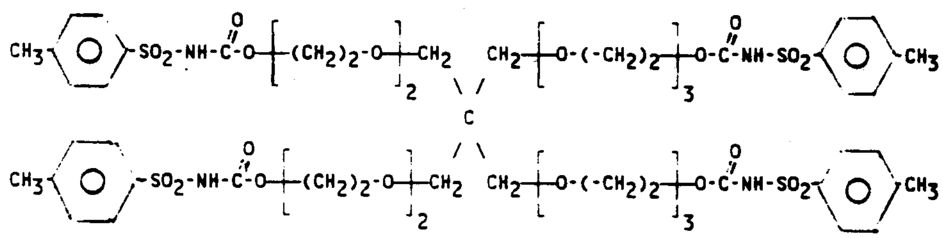

(M = 1364)

Exemple 3 Tosyluréthanne d'un polyéthertétraol du pentaérythritol de masse 356

On opère comme pour l'exemple 1 en utilisant, à la place du polyéthertriol du glycérol, 61,86 g du polyéthertétraol de masse 356 de formule suivante :

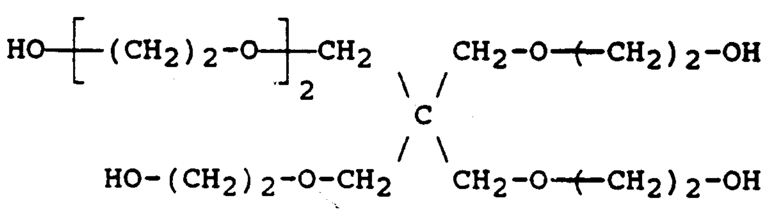

Ce polyether tétraol est un produit commercial, pouvant être obtenu par reaction entre le pentaérythritol et l'oxyde d'éthylène. Le produit utilisé a une teneur expérimentale en fonction hydroxyle de 11,01 eq/kg.

On utilise par ailleurs 135 g d'isocyanate de tosyle.

La durée totale de réaction est environ 3,25 h et la température maximale atteinte 103°C environ. A la température ambiante, le produit obtenu (196,8 g) se présente sous forme d'un solide blanc cristallisé, opaque et dur.

L'analyse par GPC montre que le produit est unique et que la réaction est quantitative.

Les spectres IR et RMN $^1$H montrent que sa structure est la suivante :

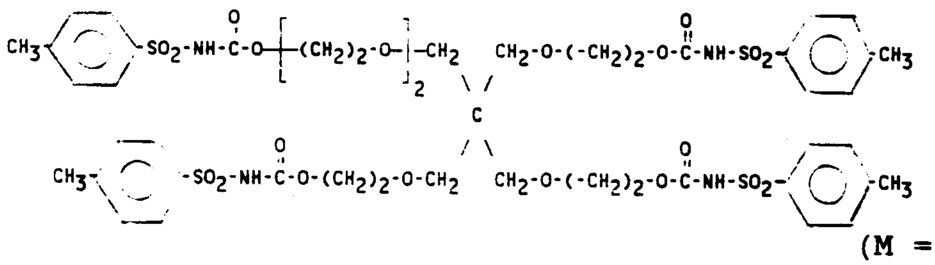

(M = 1144)

Exemple 4 Tosyluréthanne du 2-éthoxyéthanol

On opère comme pour l'exemple 1 en utilisant, a la place du polyéthertriol, 70,39 g de 2-éthoxyéthanol dont le taux d'hydroxyle est 11,09 eq/kg.

On utilise par ailleurs 154 g d'isocyanate de tosyle.

La durée totale de reaction est environ 3,5 h et la température maximale atteinte 70°C environ. A cette température, le produit obtenu (224 g) est un liquide translucide et très visqueux. A température ambiante (20°C environ), c'est un solide cristallisé, blanc, opaque et mou.

L'analyse par GPC montre que le produit est unique et que la réaction est quantitative.

Les spectres IR et RMN $^1$H montrent que sa structure est la suivante :

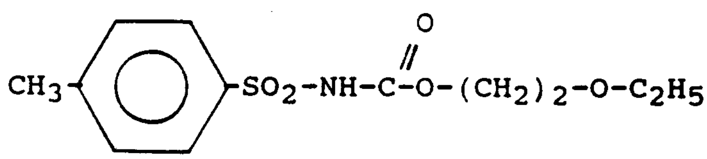

(M = 287)

Exemple 5 Tosyluréthanne du trioxyéthylèneglycol

On opère comme pour l'exemple 1 en utilisant, à la place du polyethertriol, 64,41 g de trioxyéthylèneglycol HO-[-$(CH_2)_2$-O-$]_3$H dont le taux d'hydroxyle est 13,32 eq/kg.

On utilise par ailleurs 169,2 g d'isocyanate de tosyle.

La durée totale de reaction est environ 5,5 h et la temperature maximale atteinte 95°C environ. A la température ambiante (20°C environ), le produit obtenu (233 g) se présente sous la forme d'un solide translucide, incolore, cassant et très légèrement visqueux.

L'analyse par GPC montre que le produit est unique et que la réaction est quantitative.

Les spectres IR et RMN $^1$H montrent que sa structure est la suivante :

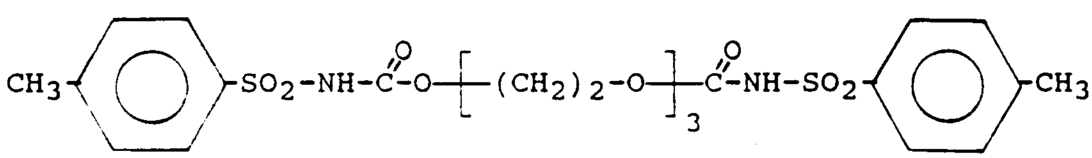

(M=544)

Exemple 6 Tosyluréthanne de l'ethanol

On opère comme pour l'exemple 1 en utilisant, à la place du polyéthertriol, 48,05 g d'ethanol dont le taux d'hydroxyle est 21,71 eq/kg.

On utilise par ailleurs 207 g d'isocyanate de tosyle.

La durée totale de réaction est environ 1,6 h et la température maximale atteinte 53°C environ. Pour couler le produit (255 g), il faut toutefois le chauffer jusqu'à 83°C environ. A la température ambiante (20°C environ), le produit obtenu est un solide blanc cristallisé.

L'analyse par GPC montre que le produit est unique et que la réaction est quantitative.

Les spectres IR et RMN $^1H$ montrent que sa structure est la suivante :

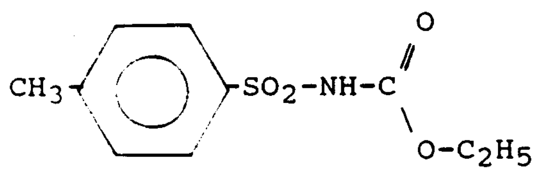

Exemple 7 Tosyluréthanne du 1,3-propanediol

On opère comme pour l'exemple 1 en utilisant, à la place du polyethertriol, 33,81 g de 1,3-propanediol dont le taux d'hydroxyle est 26,28 eq/kg.

On utilise par ailleurs 175,23 g d'isocyanate de tosyle.

La durée totale de réaction est environ 1,8 h et la température maximale atteinte environ 96°C. A cette température, le produit (209 g) est très visqueux, translucide, de couleur orange. A la température ambiante (20°C environ), c'est un solide cassant de couleur orangée, de structure :

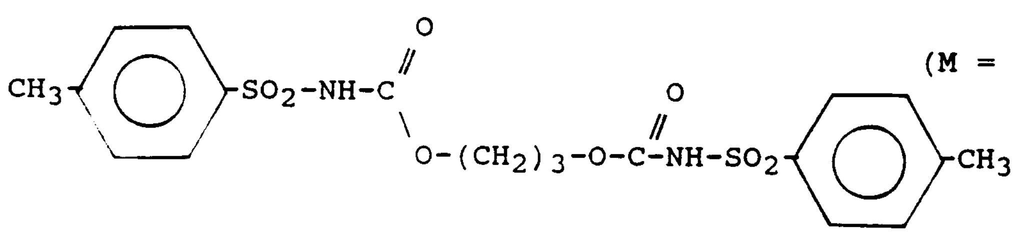

(M = 470)

Exemple 8 Tosyluréthanne du cyclohexanol

On opère comme pour l'exemple 1 en utilisant, a la place du polyéthertriol, 78,24 g de cyclohexanol dont le taux d'hydroxyle est 9,98 eq/kg.

On utilise par ailleurs 154,1 g d'isocyanate de tosyle.

La durée totale de reaction est environ 3,3 h et la température maximale atteinte 76°C. A cette température, le produit (232 g) se presente comme un liquide visqueux, translucide et incolore. A température ambiante (20°C environ), c'est un solide translucide et incolore.

L'analyse par GPC montre que le produit est unique et que la réaction est quantitative.

Les spectres IR et RMN $^1$H montrent que sa structure est la suivante :

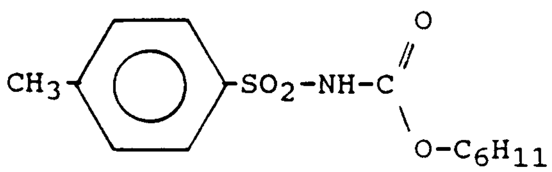

(M = 297)

Exemple 9 Tosyluréthanne du 1-octanol

On opère comme pour l'exemple 1 en utilisant, a la place du polyéthertriol, 88,91 g de $CH_3$-$(CH_2)_7$-OH dont le taux d'hydroxyle est 7,67 eq/kg.

On utilise par ailleurs 134,64 g d'isocyanate de tosyle.

La durée totale de réaction est 3,5 h environ et la température maximale atteinte 78°C. A cette température, le produit (223,5 g) se présente sous la forme d'un liquide visqueux, translucide et incolore. A température ambiante (20°C environ) le produit reste visqueux.

L'analyse par GPC montre que le produit est unique et que la réaction est quantitative.

Les spectres IR et RMN $^1$H montrent que sa structure est la suivante :

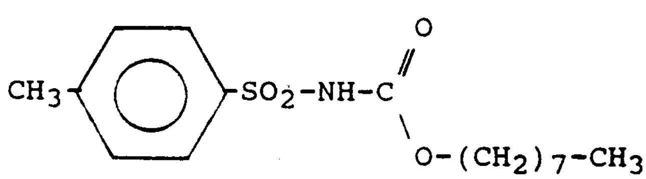

(M = 327)

Exemple 10 Tosyluréthanne du 1-pentanol

On opère comme pour l'exemple 1 en utilisant, à la place du polyéthertriol, 66,86 g de $CH_3$-$(CH_2)_4$-OH dont le taux d'hydroxyle est 11,34 eq/kg.

On utilise par ailleurs 149,6 g d'isocyanate de tosyle.

La durée totale de reaction est 3,5 h environ et la température maximale atteinte 59°C. A cette température et à température ambiante (20°C environ), le produit (216 g) se présente sous la forme d'un liquide visqueux, translucide, légèrement teinté en jaune-vert.

L'analyse par GPC montre que le produit est unique et que la réaction est quantitative.

Les spectres IR et RMN $^1$H montrent que sa structure est la suivante :

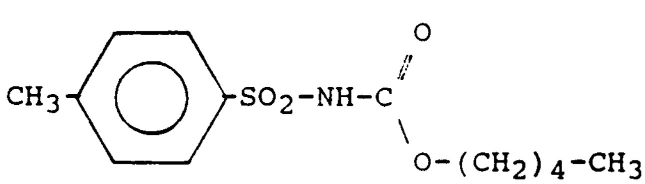

(M = 285)

Exemple 11 Tosyluréthanne de l'isobutanol

On opère comme pour l'exemple 1 en utilisant, à la place du polyéthertriol, 56,7 g de $(CH_3)_2$CH-$CH_2$OH dont le taux d'hydroxyle est 13,49 eq/kg.

On utilise par ailleurs 150,86 g d'isocyanate de tosyle.

La durée totale de réaction est 5,2 h environ et la température maximale atteinte 56°C. A cette température et à température ambiante (20°C environ), le produit (207 g) se presente sous la forme d'un liquide visqueux, translucide et incolore.

L'analyse par GPC montre que le produit est unique et que la réaction est quantitative.

Les spectres IR et RMN $^1H$ montrent que sa structure est la suivante :

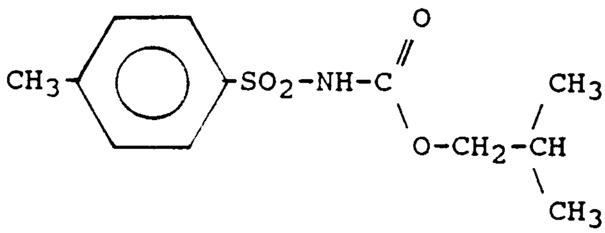

(M = 271)

Exemple 12 Tosyluréthanne de l'isopropanol

On opère comme pour l'exemple 1 en utilisant, à la place du polyéthertriol, 53,03 g de $(CH_3)_2CHOH$ dont le taux d'hydroxyle est 16,64 eq/kg.

On utilise par ailleurs 174 g d'isocyanate de tosyle.

La durée totale de réaction est 4,2 h environ et la température maximale atteinte 56°C. A cette température, le produit (227 g) est un liquide visqueux, translucide légèrement teinté en jaune-vert. A la température ambiante (20°C environ), c'est un solide cristallisé blanc et opaque.

Sa structure est :

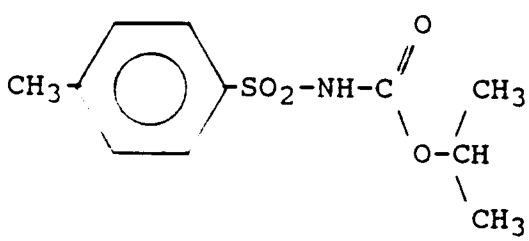

(M = 257)

Exemple 13 Tosyluréthanne de triméthylolpropane

On opère comme pour l'exemple 1 en utilisant, à la place du polyéthertriol, 99,21 g de

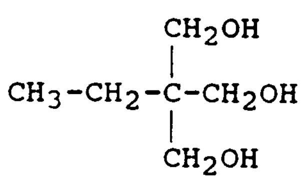

dont le taux d'hydroxyle est 22,36 eq/kg.

On utilise par ailleurs 437,44 g d'isocyanate de tosyle.

La durée totale de reaction est 3,8 h environ et la temperature maximale atteinte 138°C. A cette température, le produit (536 g) est très visqueux. A la temperature ambiante (20°C environ), c'est un solide cassant et opaque, de couleur légèrement jaune-orangée.

L'analyse par GPC montre que le produit est unique et que la reaction est quantitative.

Les spectres IR et RMN $^1H$ montrent que sa structure est la suivante :

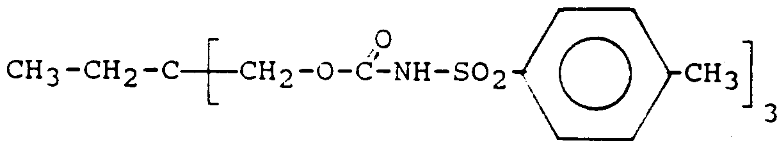

(M = 725)

Exemple 14 Tosyluréthanne du 2-butanol

On opère comme pour l'exemple 1 en utilisant, a la place du polyethertriol, 65,99 g de $CH_3$-$CH_2$-CHOH-$CH_3$ dont le taux d'hydroxyle est 13,49 eq/kg.

On utilise par ailleurs 175,58 g d'isocyanate de tosyle.

La duree totale de réaction est 4,3 h environ et la température maximale atteinte 53°C. A cette température et a la temperature ambiante (20°C environ), le produit (241 g) se présente sous forme d'un liquide visqueux, translucide, légèrement teinté en jaune-vert.

L'analyse par GPC montre que le produit est unique et que la réaction est quantitative.

Les spectres IR et RMN $^1H$ montrent que sa structure est :

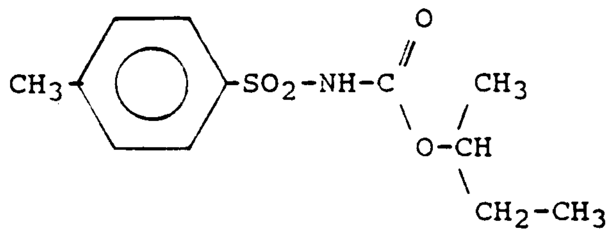

(M = 271)

Exemple 15 Tosyluréthanne du 3-méthyl 2-butanol

On opère comme pour l'exemple 1 en utilisant, à la place du polyéthertriol, 88,15 g de $CH_3$-CHOH-CH($CH_3$)$_2$ dont le taux d'hydroxyle est 11,34 eq/kg.

On utilise par ailleurs 197,21 g d'isocyanate de tosyle.

La durée totale de réaction est 4 h environ et la température maximale atteinte 90°C. A cette température, le produit est visqueux et l'on peut aisément le couler. A la température ambiante (20°C environ), c'est un solide blanc cristallisé.

L'analyse par GPC montre que le produit est unique et que la réaction est quantitative.

Les spectres IR et RMN $^1H$ montrent que sa structure est :

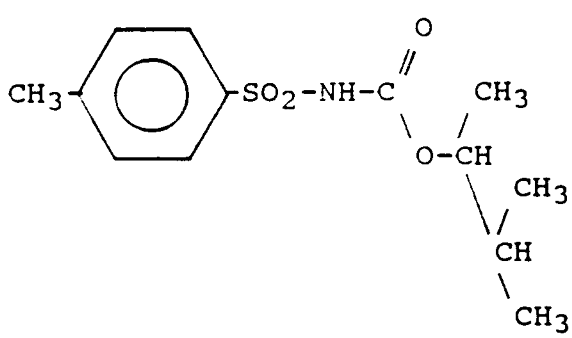

(M = 285)

Exemple 16 Tosyluréthanne du glycérol

On opère comme pour l'exemple 1 en utilisant, à la place du polyethertriol, 31,32 g de glycérol dont le taux d'hydroxyle est 32,57 eq/kg.

On utilise par ailleurs 200 g d'isocyanate de tosyle.

La durée totale de réaction est 4,3 h environ et la température maximale atteinte 135°C. A cette température, le produit est un liquide très visqueux, opaque et blanc. A la température ambiante (20°C environ), c'est un solide cristallisé, cassant, légèrement jaunâtre.

L'analyse par GPC montre que le produit est unique et que la reaction est quantitative.

Les spectres IR et RMN $^1H$ montrent que sa structure est :

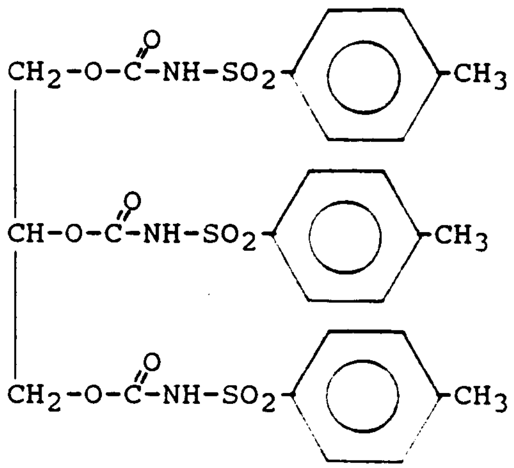

(M = 683)

Exemple 17 Tosyluréthanne du xylitol

On opère comme pour l'exemple 1 en utilisant, à la place du polyéthertriol, 50 g (0,332 mol) de xylitol : $CH_2OH-(CHOH)_3-CH_2OH$.

On utilise par ailleurs 330 g (1,67 mol) d'isocyanate de tosyle.

La durée totale de reaction est 3,3 h environ et la temperature maximale atteinte est superieure à 120°C.

Le produit obtenu (380 g) est un solide dur et cassant à la température ambiante.

Sa structure est :

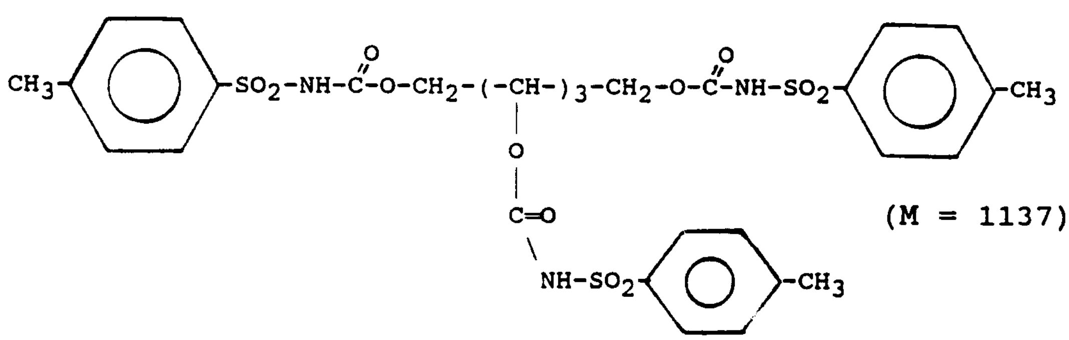

(M = 1137)

Exemples 18 à 34 Vernis à ongles nitrocellulosiques selon l'invention

Les vernis des exemples 18 à 34 ont été respectivement réalisés avec, comme résine garnissante, les tosyluréthannes des exemples 1 à 17. On realise tout d'abord, par simple mélange des constituants, une base incolore non thixotrope dont la composition pondérale est la suivante :

| | |
|---|---|
| Nitrocellulose sèche type E28 | 12 % |
| Camphre | 1,5 % |
| Phtalate de dibutyle | 7 % |
| Résine garnissante | 11,5 % |
| Isopropanol | 6 % |
| Acétate d'éthyle | 14 % |
| Acétate de butyle | 25 % |
| Toluène | 23 % |

On realise ensuite, par simple mélange des constituants, un gel nitrocellulosique thixotrope incolore dont la composition ponderale est la suivante :

| | |
|---|---|
| Nitrocellulose sèche type E33 | 15 % |
| Camphre | 3 % |
| Isopropanol | 13 % |
| Acétate d'éthyle | 5 % |
| Acétate de butyle | 25 % |
| Toluène | 32 % |
| Diméthylbenzyldodécylammonium montmorillonite | 7 % |

On réalise alors une base antisédimentante nitrocellulosique thixotrope incolore, qui est un vernis à ongles incolore, par simple mélange des constituants suivants :

- 20,00 parties en poids du gel nitrocellulosique thixotrope incolore précité,
- 0,03 partie en poids d'acide phosphorique à 85 %,
- 79,97 parties en poids de la base incolore non thixotrope correspondant à chaque exemple.

La composition pondérale de ces 17 vernis à ongles incolores est donc la suivante :

| | |
|---|---|
| Nitrocellulose sèche | 12,60 % |
| Camphre | 1,80 % |
| Phtalate de dibutyle | 5,60 % |
| Résine garnissante | 9,20 % |
| Isopropanol | 7,40 % |
| Acétate d'éthyle | 12,20 % |
| Acétate de butyle | 24,99 % |
| Toluène | 24,79 % |
| Diméthylbenzyldodécylammonium montmorillonite | 1,40 % |
| $H_3PO_4$ (85%) | 0,03 % |

Les caractéristiques de ces vernis à ongles incolores sont regroupées dans le tableau suivant, qui présente également les résultats obtenus pour un vernis à ongles incolore de l'état de la technique, réalisé comme selon l'exemple 18, mais en remplaçant la résine garnissante selon l'invention par la résine arylsulfonamideformol commercialisée sous le nom de SANTOLITE (exemple comparatif).

| Caractéristiques / Ex N° | Viscosité Brookfield à 20°C - Plongeur n°3 5 tr/min | 50 tr/min | 5 tr/min | Indice de thixotropie | Dureté "Persoz" (3h) | Brillance "Gardner" à 60° | Taux de formol libérable |
|---|---|---|---|---|---|---|---|
| 18 | 2 300 | 960 | 1 500 | 1,6 | / | 82 | < 0,01 % |
| 19 | 2 140 | 900 | 1 440 | 1,6 | / | / | < 0,01 % |
| 20 | 2 300 | 914 | 1 500 | 1,6 | 87 | 82 | < 0,01 % |
| 21 | 1 800 | 670 | 1 100 | 1,6 | / | 84 | < 0,01 % |
| 22 | 1 640 | 710 | 900 | 1,3 | 78 | 83 | < 0,01 % |
| 23 | 1 340 | 652 | 820 | 1,3 | / | 84 | < 0,01 % |
| 24 | 1 540 | 768 | 980 | 1,3 | 103 | 84 | < 0,01 % |
| 25 | 1 340 | 652 | 900 | 1,4 | 99 | 84 | < 0,01 % |
| 26 | 1 320 | 636 | 900 | 1,4 | / | 82 | < 0,01 % |
| 27 | 1 360 | 634 | 900 | 1,4 | / | 84 | < 0,01 % |
| 28 | 1 500 | 644 | 900 | 1,4 | 86 | 82 | < 0,01 % |
| 29 | 1 400 | 650 | 900 | 1,4 | / | 83 | < 0,01 % |
| 30 | 4 300 | 1 320 | 1 900 | 1,4 | / | 85 | < 0,01 % |
| 31 | 2 200 | 940 | 1 400 | 1,5 | / | 83 | < 0,01 % |
| 32 | 2 180 | 920 | 1 300 | 1,4 | / | 83 | < 0,01 % |
| 33 | 3 180 | 1 300 | 1 800 | 1,4 | / | 86 | < 0,01 % |
| 34 | 2 040 | 720 | 1 100 | 1,5 | 180 | 87 | < 0,01 % |
| Comparatif | 3 100 | 930 | 1 680 | 1,8 | 120 | 80 | 0,60 % |

Pour déterminer l'indice de thixotropie, on mesure tout d'abord la viscosité Brookfield après agitation pendant 1 min à 5 tr/min. On agite de nouveau 1 min, mais à 50 tr/min, puis on effectue une seconde mesure. On agite de nouveau 1 min à 5 tr/min, puis on effectue une troisième mesure. L'indice de thixotropie est le rapport entre les valeurs respectivement obtenues aux 3$^{ème}$ et 2$^{ème}$ mesures.

Pour déterminer la dureté "Persoz", on applique un film d'épaisseur 150µm du vernis sur une plaque de verre puis on réalise les mesures avec un pendule de Persoz après divers temps de séchage à 20°C et sous hygrométrie de 65 %. Les valeurs indiquées sont celles obtenues après 3 h de séchage.

Pour déterminer la brillance "Gardner", on applique un film de vernis d'épaisseur 200µm sur un support plan, puis on mesure, après sechage superieur a 1 h à 20°C, à l'aide d'un brillancemètre, le pourcentage de lumière emise par une source sur le film de vernis selon un angle incident de 60° et réémise par le film selon ce même angle. Plus la quantité de lumière réémise est importante, plus le film de vernis est brillant.

Pour déterminer le taux de formol libérable, on réalise tout d'abord une solution à environ 100 g/l de résine dans l'acetone, puis on hydrolyse la résine à 80°C environ après ajout d'une solution aqueuse d'acide chlorhydrique dilue.

On distille ensuite le formol libéré, puis on le fait reagir avec l'acétylacétone en présence d'une solution aqueuse d'acétate d'ammonium. On dose alors le dérivé pyridinique coloré forme par spectrophotometrie à la longueur d'onde de 413 nm.

Pour les exemples 18 à 29 et 34, ainsi que pour l'exemple comparatif, on a ensuite realise des vernis à ongles colores en ajoutant, dans les vernis à ongles incolores, 1,05 % en poids de $TiO_2$ et 0,036 % en poids du pigment organique rouge "DC Red 34" selon l'appellation de la Food and Drug Administration (FDA).

Le vernis à ongles coloré des exemples 23, 25 à 29, 34 et de l'exemple comparatif est ensuite appliqué sur l'ongle (2 couches) par une manucure qui vérifie l'état des vernis au cours du temps (aspect general, écaillage, usure...). Les personnes dont les ongles sont soumis à ce test doivent avoir une activité normale et habituelle. Le résultat, exprimé en nombre de jours pour lesquels le vernis est jugé avoir un aspect général satisfaisant, est le suivant :

| | |
|---|---|
| Exemple 23 | 2,0 j |
| Exemple 25 | 2,1 j |
| Exemple 26 | 2,0 j |
| Exemple 27 | 2,3 j |
| Exemple 28 | 2,0 j |
| Exemple 29 | 2,4 j |
| Exemple 34 | 2,4 j |
| Exemple comparatif | 2,3 j |

De plus, la manucure a noté, d'une part que le temps de séchage (temps à partir duquel un doigt ne laisse plus d'empreinte sur le vernis de l'ongle) est nettement amélioré par rapport à l'exemple comparatif (3 à 7 min pour les exemples selon l'invention au lieu de 8 min) et d'autre part que la brillance (appréciation visuelle) est, pour les exemples selon l'invention, supérieure à celle obtenue pour l'exemple comparatif.

Par ailleurs, on a réalisé, avec les vernis à ongles colorés des exemples 18 a 29 et 34, ainsi que pour le vernis à ongles coloré de l'exemple comparatif, un test de sédimentation, d'une part a 20°C et d'autre part à 50°C, dans des flacons du type de ceux dans lesquels les vernis à ongles sont usuellement conditionnes et commercialises. Les contrôles visuels périodiques montrent, aussi bien à 20°C qu'à 50°C, pour tous les exemples selon l'invention ainsi que pour l'exemple comparatif, qu'aucun dépôt ne s'est formé au fond des flacons après 4 mois de stockage.

**Revendications**

1. Poly(arylsulfonyluréthannes), caractérisés en ce qu'ils comprennent de 2 à 5 groupements arylsulfonyluréthannes et en ce que leur masse moléculaire est comprise entre 450 et 1500.

2. Poly(arylsulfonyluréthannes) selon la revendication 1, caractérisés en ce que les groupements arylsulfonyluréthannes sont des groupements paratolylsulfonyluréthannes.

3. Poly(arylsulfonyluréthannes) selon l'une quelconque des revendications précédentes, caractérisés en ce qu'ils répondent à la formule générale (II)

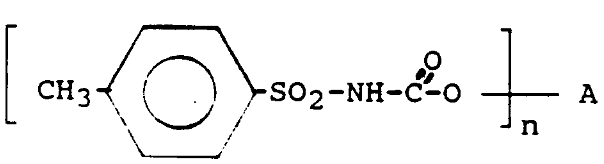

dans laquelle n représente un nombre entier tel que 2 ≦ n ≦ 5 et A représente un radical organique, di, tri, tétra ou pentavalent, selon que n est respectivement égal à 2, 3, 4 ou 5, qui est le squelette de polyols de structure $A(OH)_n$ choisis dans le groupe constitué par les polyméthylèneglycols, les polyoxyalkylèneglycols, les polyéthers triols, les polyéthers tétraols, le glycérol, les triméthylolalcanes et le xylitol.

4. Poly(arylsulfonyluréthannes) selon la revendication 3, caractérisés en ce que les polyéthers triols répondent aux formules générales :

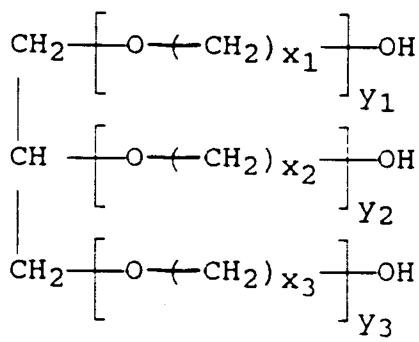

ou

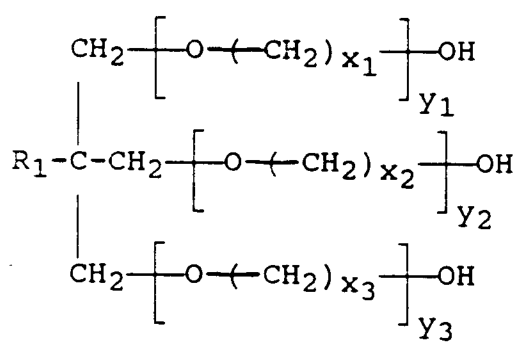

dans lesquelles

- . $x_1$, $x_2$ et $x_3$, identiques ou différents, de préférence identiques, représentent 1, 2, 3 ou 4,
- . $y_1$, $y_2$ et $y_3$, identiques ou différents, représentent 0, 1, 2, 3, 4, 5 ou 6, à la condition que $y_1$, $y_2$ et $y_3$ ne soient pas simultanément nuls,
- . $R_1$ représente l'hydrogène ou une chaîne alkyle, linéaire ou ramifiée, comportant 1 à 8 atomes de carbone, ou une chaine alkyle comportant 1 à 8 atomes de carbone substituée par au moins un radical alcoxy,

et les polyéthers tétraols répondent à la formule générale

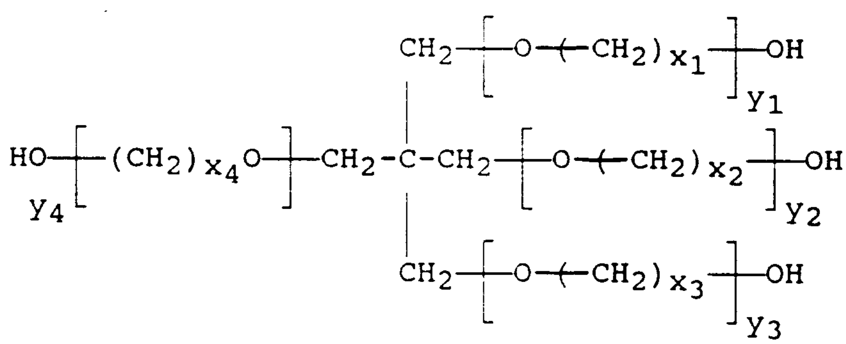

dans laquelle

- . $x_1$, $x_2$, $x_3$ et $x_4$, identiques ou différents, de préférence identiques, représentent 1, 2, 3 ou 4,
- . $y_1$, $y_2$, $y_3$ et $y_4$, identiques ou différents, représentent 0, 1, 2, 3, 4, 5 ou 6, à la condition que $y_1$, $y_2$, $y_3$ et $y_4$ ne soient pas simultanément nuls.

5. Vernis à ongles essentiellement constitué de nitrocellulose, d'une résine garnissante, d'un plastifiant, d'un agent gélifiant et d'un système solvant-diluant, caractérisé en ce que la résine garnissante comprend un arylsulfonyluréthanne.

6. Vernis à ongles selon la revendication 5, caractérisé en ce que l'arylsulfonyluréthanne est un poly(arylsulfonyluréthanne) selon l'une quelconque des revendications 1 à 4.

7. Vernis à ongles selon l'une quelconque des revendications 5 et 6, caractérisé en ce qu'il est constitué de 5 % à 20 % en poids de nitrocellulose, de 1 % à 20 % en poids d'une résine arylsulfonyluréthanne, de 0,1 % à 5 % en poids d'argile organophile de type montmorillonite modifiée par une amine, de 0,1 % à 20 % en poids de plastifiant, de 5 % à 85 % en poids d'un système solvant-diluant, de 0 à 10 % en poids de pigment ou colorant et de 0 à 10 % en

poids d'additifs usuels, de préférence un agent mouillant de l'argile organophile et/ou un agent gonflant, la somme des pourcentages étant égale à 100.

**8.** Utilisation d'un arylsulfonyluréthanne comme résine garnissante dans les vernis à ongles nitrocellulosiques.

## Claims

**1.** Poly(arylsulphonylurethanes), characterized in that they comprise 2 to 5 arylsulphonylurethane groups and in that their molecular weight is between 450 and 1500.

**2.** Poly(arylsulphonylurethanes) according to claim 1, characterized in that the arylsulphonylurethane groups are paratolylsulphonylurethane groups.

**3.** Poly(arylsulphonylurethanes) according to either of the preceding claims, characterized in that they correspond to the following general formula (II)

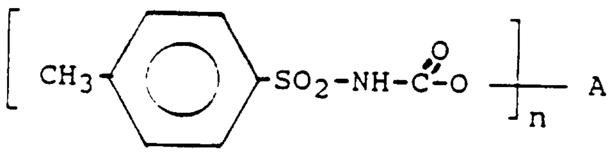

in which n represents a whole number such that $2 \leq n \leq 5$ and A represents a di, tri, tetra or pentavalent organic radical, according to which n is equal to 2, 3, 4 or 5 respectively, which is the skeleton of polyols of the structure $A(OH)_n$ selected from the group consisting of polymethyleneglycols, polyoxyalkyleneglycols, polyether triols, polyether tetraols, glycerol, trimethylolalkanes and xylitol.

**4.** Poly(arylsulphonylurethanes) according to claim 3, characterized in that the polyether triols correspond to the general formulae :

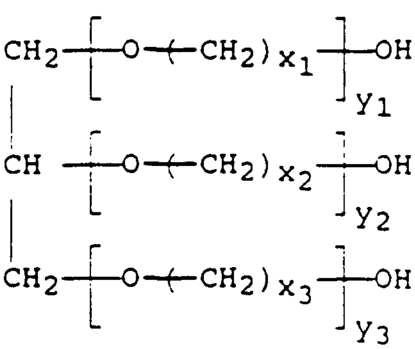

ou

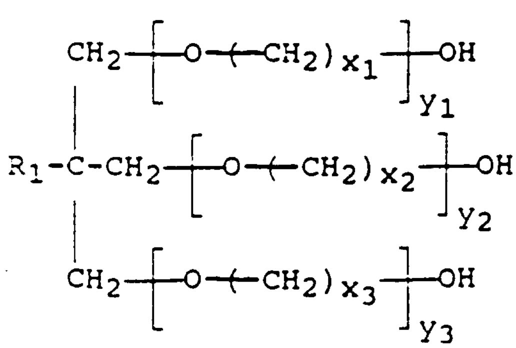

in which

- $x_1$, $x_2$ and $x_3$, identical or different, preferably identical, represent 1, 2, 3 or 4,
- $y_1$, $y_2$ and $y_3$, identical or different, represent 0, 1, 2, 3, 4, 5 or 6, on condition that $y_1$, $y_2$ and $y_3$ are not simultaneously zero,
- $R_1$ represents hydrogen or a linear or branched alkyl chain, having 1 to 8 carbon atoms, or an alkyl chain having 1 to 8 carbon atoms substituted with at least one alkoxy radical,

and the polyether tetraols correspond to the general formula

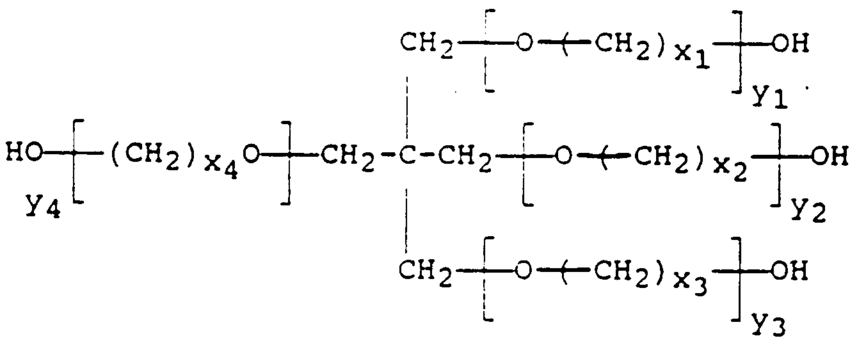

in which

- $x_1$, $x_2$, $x_3$ and $x_4$, identical or different, preferably identical, represent 1, 2, 3 or 4,
- $y_1$, $y_2$, $y_3$ and $y_4$, identical or different, represent 0, 1, 2, 3, 4, 5 or 6, on condition that $y_1$, $y_2$, $y_3$ and $y_4$ are not simultaneously zero.

**5.** Nail varnish consisting essentially of nitrocellulose, a high-build resin, a plasticizer, a gelling agent and a solvent-diluent system, characterized in that the high-build resin comprises an arylsulphonylurethane.

**6.** Nail varnish according to claim 5, characterized in that the arylsulphonylurethane is a poly(arylsulphonylurethane) according to any one of claims 1 to 4.

**7.** Nail varnish according to either of claims 5 or 6, characterized in that it consists of 5 % to 20 % by weight of nitrocellulose, 1 % to 20 % by weight of an arylsulphonylurethane resin, 0.1 % to 5 % by weight of an organophilic clay of the montmorillonite type modified with an amine, 0.1 % to 20 % by weight of plasticizer, 5 % to 85 % by weight of a solvent-diluent system, 0 % to 10 % by weight of a pigment or colorant and 0 % to 10 % by weight of the usual additives, preferably an agent for wetting the organophilic clay and/or a swelling agent, the sum of the percentages being equal to 100.

**8.** Use of an arylsulphonylurethane as a high-build resin in nitrocellulose nail varnishes.

**Patentansprüche**

**1.** Poly(arylsulfonylurethane),
dadurch gekennzeichnet, daß
sie 2 bis 5 Arylsulfonylurethan-Gruppen enthalten und ihre Molekülmasse im Bereich von 450 bis 1500 liegt.

**2.** Poly(arylsulfonylurethane) nach Anspruch 1,
dadurch gekennzeichnet, daß
die Arylsulfonylurethan-Gruppen p-Toluolsulfonylurethan-Gruppen sind.

**3.** Poly(arylsulfonylurethane) nach einem der vorhergehenden Ansprüche,
dadurch gekennzeichnet, daß
sie der allgemeinen Formel (II)

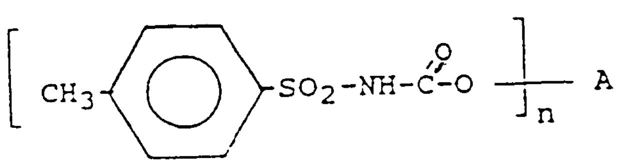

entsprechen,
worin bedeuten:

n eine ganze Zahl im Bereich von $2 \leq n \leq 5$ und
A eine je nachdem, ob n = 2, 3, 4 bzw. 5 ist, zwei-, drei, vier- oder fünfwertige organische Gruppe, die das Gerüst eines Polyols der Struktur $A(OH)_n$ darstellt und unter den Polymethylenglykolen, Polyoxyalkylenglykolen, den Polyethern mit 3 OH-Gruppen, den Polyethern mit 4 OH-Gruppen, Glycerin, den Trimethylolalkanen und Xylit ausgewählt ist.

**4.** Poly(arylsulfonylurethane) nach Anspruch 3,
dadurch gekennzeichnet, daß
die Polyether mit 3 OH-Gruppen die nachstehenden allgemeinen Formeln aufweisen:

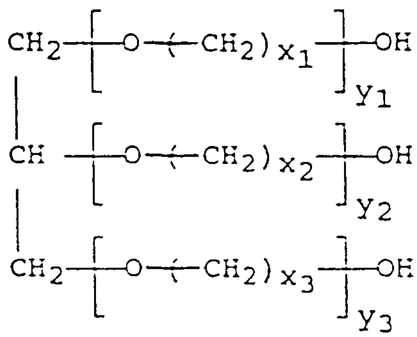
oder
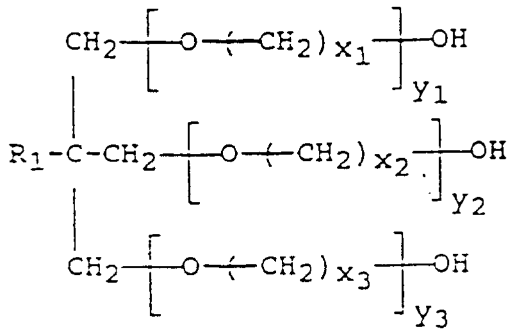
,

worin bedeuten:

- $x_1$, $x_2$ und $x_3$, die identisch oder voneinander verschieden und vorzugsweise identisch sind, 1, 2, 3 oder 4,
- $y_1$, $y_2$ und $y_3$, die identisch oder voneinander verschieden sind, 0, 1, 2, 3, 4, 5 oder 6, mit der Maßgabe, daß $y_1$, $y_2$ und $y_3$ nicht gleichzeitig Null sind,
- $R_1$ Wasserstoff oder eine geradkettige oder verzweigte Alkylkette mit 1 bis 8 Kohlenstoffatomen oder eine Alkylkette mit 1 bis 8 Kohlenstoffatomen, die mit mindestens einem Alkoxyradikal substituiert ist,

und die Polyether mit 4 OH-Gruppen die allgemeine Formel aufweisen:

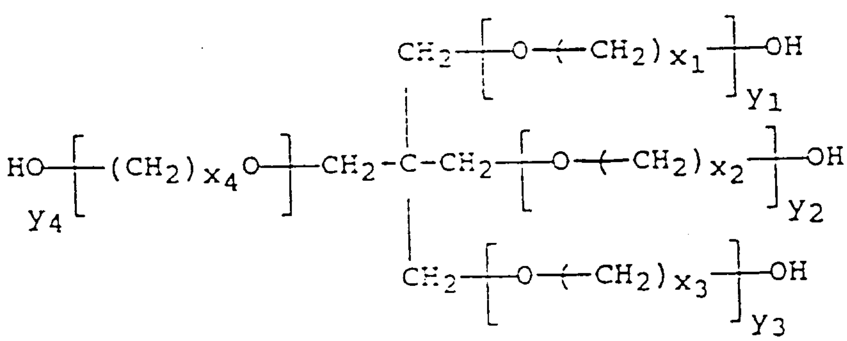
,

worin bedeuten:

- $x_1$, $x_2$, $x_3$ und $x_4$, die identisch oder voneinander verschieden und vorzugsweise identisch sind, 1, 2, 3 oder 4,
- $y_1$, $y_2$, $y_3$ und $y_4$, die identisch oder voneinander verschieden sind, 0, 1, 2, 3, 4, 5 oder 6, mit der Maßgabe, daß $y_1$, $y_2$, $y_3$ und $y_4$ nicht gleichzeitig Null sind.

**5.** Nagellack, der im wesentlichen aus Cellulosenitrat, einem Füllharz, einem Weichmacher, einem Geliermittel und einem Lösungsmittel-Verdünnungsmittel-System besteht,

dadurch gekennzeichnet, daß das Füllharz ein Arylsulfonylurethan umfaßt.

**6.** Nagellack nach Anspruch 5, dadurch gekennzeichnet, daß das Arylsulfonylurethan ein Poly(arylsulfonylurethan) nach einem der Ansprüche 1 bis 4 ist.

**7.** Nagellack nach Anspruch 5 oder 6, dadurch gekennzeichnet, daß er aus 5 bis 20 Gew.-% Cellulosenitrat, 1 bis 20 Gew.-% Arylsulfonylurethanharz, 0,1 bis 5 Gew.-% organophilem Ton vom Typ eines mit einem Amin modifizierten Montmorillonits, 0,1 bis 20 Gew.-% Weichmacher, 5 bis 85 Gew.-% eines Lösungsmittel-Verdünnungsmittel-Systems, 0 bis 10 Gew.-% Pigment oder Farbstoff und 0 bis 10 Gew.-% üblichen Additiven, vorzugsweise einem Netzmittel für den organophilen Ton und/oder ein Quellmittel, besteht, wobei die Summe der Prozentanteile gleich 100 ist.

**8.** Verwendung eines Arylsulfonylurethans als Füllharz in Cellulosenitrat-Nagellacken.